(19) Europäisches Patentamt
European Patent Office
Office européen des brevets

(11) Veröffentlichungsnummer : **0 146 097 B2**

(12) **NEUE EUROPÄISCHE PATENTSCHRIFT**

(45) Veröffentlichungstag der neuen Patentschrift : **06.05.92 Patentblatt 92/19**

(51) Int. Cl.$^5$ : **C07C 213/00,** C07C 217/00, F15D 1/00, F17D 1/16

(21) Anmeldenummer : **84114979.2**

(22) Anmeldetag : **08.12.84**

# (54) Oxalkylierte quaternäre Ammonium-Verbindungen, Verfahren zu deren Herstellung und deren Verwendung alsStrömungsbeschleuniger.

(30) Priorität : **17.12.83 DE 3345806**

(43) Veröffentlichungstag der Anmeldung : **26.06.85 Patentblatt 85/26**

(45) Bekanntmachung des Hinweises auf die Patenterteilung : **08.06.88 Patentblatt 88/23**

(45) Bekanntmachung des Hinweises auf die Entscheidung über den Einspruch : **06.05.92 Patentblatt 92/19**

(84) Benannte Vertragsstaaten : **BE CH DE FR GB LI NL SE**

(56) Entgegenhaltungen :
**EP-A- 0 091 086**
**EP-A- 0 098 802**

(56) Entgegenhaltungen :
**DE-B- 1 619 043**
**SU-A- 433 177**
**US-A- 3 223 718**
**US-A- 3 223 718**
**US-A- 3 807 983**
**CHEMICAL ABSTRACTS, Band 101, Nr. 22, 26. November 1984, Seite 80, Nr. 193617s, Columbus, Ohio, US**

(73) Patentinhaber : **HOECHST AKTIENGESELLSCHAFT**
**Postfach 80 03 20**
**W-6230 Frankfurt am Main 80 (DE)**

(72) Erfinder : **Ohlendorf, Dieter, Dr.**
**Am Kühlen Grund 4**
**W-6237 Liederbach (DE)**
Erfinder : **Hofinger, Manfred, Dr.**
**Hoher Göll Weg 7**
**W-8269 Burgkirchen (DE)**

EP 0 146 097 B2

Jouve, 18, rue Saint-Denis, 75001 PARIS

**Beschreibung**

Es ist allgemein bekannt, daß turbulent strömende Flüssigkeiten an den sie begrenzenden Wänden einen Reibungswiderstand erfahren. Es ist auch bekannt, daß man diesen Reibungswiderstand durch Zusatz geringer Mengen bestimmter Stoffe herabsetzen kann. Stoffe, die diese Wirkung zeigen, werden im englischen Sprachgebrauch als "drag reducing agents" bezeichnet. Im deutschsprachigen Raum wird für diese Stoffe die Bezeichnung "Strömungsbeschleuniger" verwendet (im folgenden abgekürzt durch SB). Unter einem Strömungsbeschleuniger versteht man danach einen Stoff, der in geringerer Menge einer turbulent oder pulsierend strömenden Flüssigkeit zugesetzt, diese Flüssigkeit - unter sonst gleichen Bedingungen - schneller strömen läßt. Strömungsbeschleuniger bewirken, daß man mit einer gegebene Pumpe durch eine gegebene Rohrleitung mehr Flüssigkeit fördern kann.

In vielen Fällen ist allein schon diese Tatsache ein technischer Gewinn, wenn z. B. eine Rohrleitung im Normalbetrieb voll ausgelastet ist und zu bestimmten Zeiten ein Spitzenvorbrauch zu fördern wäre. Da mit einer gegebenen Pumpleistung bei der Verwendung von Strömungsbeschleunigern mehr Flüssigkeit gefördert werden kann, wird auch in vielen Fällen die damit verbundene Energieeinsparung einen technischen Vorteil bringen. Schließlich kann man, wenn man die Durchsatzmenge nicht vergrößern will, bei Verwendung von SB den Druckverlust herabsetzen oder Rohre mit kleinerem Querschnitt verwenden. Beides sind Maßnahmen, die die Wirtschaftlichkeit im Betrieb einer Rohrleitung verbessern können.

Als Strömungsbeschleuniger für Wasser oder wäßrige Lösungen sind neben hochmolekularen Verbindungen, wie Polyethylenoxid und Polyacrylamid, Lösungen von einigen Tensiden bekannt. Zusätze hochmolekularer Verbindungen besitzen jedoch nur eine beschränkte praktische Einsatzfähigkeit als Strömungsbeschleuniger, da sie in Bereichen hoher Scher- und Dehnbeanspruchung, wie z. B. in Pumpen oder in geringem Maß in der turbulenten Grenzschicht nahe der Rohrwandung, durch mechanischen Abbau irreversibel ihre Wirksamkeit als Strömungbeschleuniger verlieren. Für geschlossene Wasserkreisläufe, in denen die selbe wäßrige Lösung durch ein Rohrleitungsystem ständig umgepumpt wird, sind hochmolekulare Zusätze folglich ungeeignet, da der irreversible mechanische Abbau eine laufende Nachdosierung mit wirksamer hochmolekularer Substanz erforderlich macht.

Zusätze von Tensiden in Wasser weisen bekanntlich nicht den Nachteil des irreversiblen mechanischen Abbaues auf (US-PS-3 961 639). Zwar läßt sich auf hier in Bereichen sehr hoher Dehn- und Scherbeanspruchung, wie z. B. in Pumpen, ein mechanischer Abbau beobachten, der jedoch völlig reversibel ist, sobald die Lösung diese Bereiche passiert hat. So die strömungsbeschleunigende Wirkung einer wäßrigen Lösung von Na-Oleat bei Zusatz von KCl + KOH oder NaOH von Savins beschrieben (Reol. Acta 6, 323 (1967)). Asslanow et al., Akad. Nauk SSSR, Mekh. Zhidk. Gaza 1, 36-43 (1980)) untersuchten u.a. wäßrige Lösungen von Na-Laurat, -Myristat, -Palmitat und -Stearat bei pH = 11 als SB.

Chang et al. (US-PS-3 961 639) beschrieben die strömungsbeschleunigende Wirkung wäßriger Lösungen einiger nicht ionischer Tenside mit Fremdelektrolytzusatz bei Temperaturen im Bereich des Trübungspunktes.

Wesentliche Nachteile der genannten Tensidlösungen sind ihre relativ hohen Einsatzkonzentrationen von mindestens 0,25 Gew.-%, die Bildung unlöslicher Seifen mit $Ca^{2+}$ und anderen Kationen, die Ausbildung zweier Phasen, die sich bei längerem Stehen trennen und zu Verstopfungen führen können, die Notwendigkeit der Zugabe von korrosionsfördernden Fremdelektrolyten, sowie ein sehr enger Temperaturbereich von wenigen Grad Celsius, in dem SB-Wirkung auftritt. Ebenfalls bekannt ist, daß wäßrige Lösungen einiger kationischer Tenside, wie z. B. Cetylpyridiniumbromid (Inzh. Fiz. Zh. 38, No. 6, 1031-1037 (1980)) oder Cetyltrimethylammoniumbromid (Nature, 214, 585-586 (1967)) in jeweils 1 : 1 molarer Mischung mit α-Naphthol als SB wirksam sind.

Derartige Mischungen verlieren jedoch ihre Wirksamkeit als SB innerhalb weniger Tage durch chemischen Abbau (US-PS-3 961 639; J.L. Jakin, J.L. Chang S. DI-1 bis DI-4 Conference Proceeding: Intern. Conference on Dra Reduction, 4. - 6. 9. 1974 Rolla, Missouri, USA). Als weiterer entscheidender Nachteil dieser Mischungen ist die schlechte Wasserlöschkeit des α-Naphthols zu nennen, sowie insbesondere der sehr hohe Anteil an korrosiven Halogenidionen wie z. B. das $Br^-$. Bekannt ist auch die Verwendung von n-Hexadecyltrimethylammoniumsalicylat als SB. Diese Verbindung läßt sich jedoch nur durch aufwendige Reinigungsoperationen frei von korrosiven Halogen-Ionen herstellen und zeigt zudem eine Wirksamkeit als SB nur bis zu einer Temperatur von 70°C. Die Verwendung verschiedener quaternärer Ammoniumverbindungen als SB ist auch aus der Anmeldung Wo 83/01583 bekannt.

Überraschenderweise wurde nun gefunden, daß zum Unterschied zu allen bisher als SB bekannten Tensiden die nachfolgend aufgeführten Verbindungen in reiner Form auch ohne jegliche Zusätze in wäßriger Lösung schon in kleinsten Konzentrationen als Strömungsbeschleuniger wirksam sind, wobei diese Tenside, bedingt durch das Herstellverfahren, völlig frei von korrosiven Elektrolyten, wie z. B. $Cl^-$ oder $Br^-$, sind. Weiterhin wurde gefunden, daß diese Verbindungen ihre Wirksamkeit auch bei Dauerbeanspruchung über Wochen nicht

verlieren. Zusätzlich zeigen einige der Verbindungen auch Wirksamkeit über 80°C.

Gegenstand der Erfindung sind neue quaternäre Ammoniumverbindungen gemäß Anspruch 1.

Die erfindungsgemäßen Ammoniumverbindungen werden hergestellt, ausgehend von tertiären Aminen der Formel

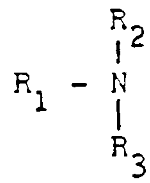

Diese wiederum sind in bekannter Weise erhältlich durch reduktive Alkylierung von primären Aminen mit Formaldehyd. Die klassische Reaktionsweise dabei ist die Leuckart-Wallach-Reaktion unter Verwendung von Ameisensaüre als Reduktionsmittel. Bei moderneren Verfahren wird die reduktive Alkylierung mit Formaldehyd in Gegenwart von Nickelkatalysatoren unter Wasserstoffdruck durchgeführt. Zusätzliche Möglichkeiten zur Herstellung dieser unsymmetrischen tertiären Amine sind die Reaktion von Fettalkoholen mit Dimethylamin in Gegenwart von Kupfer-, Chrom- oder Kobaltkatalysatore sowie die Umsetzungen von Fettnitrilen, Alkylhalogeniden, Alkanalen oder Fettsäuren mit Dimethylamin.

Das tertiäre Amin wird nun mit der Säurekomponente und mit Ethylenoxid umgesetzt. Dabei können diese beiden Reaktionspartner gleichzeitig zugegeben und zur Umsetzung gebracht werden. Bevorzugt ist es jedoch, erst die Neutralisation mit der jeweiligen Säure vorzunehmen, was während des Aufheizens auf die Reaktionstemperatur geschehen kann, und danach Ethylenoxid zuzusetzen. Die Carbonsäure wird in äquimolarer Menge zum tertiären Amin eingesetzt, die Menge des Ethylenoxids beträgt 1,5 bis 5 mol, vorzugsweise ca. 2 mol pro mol Amin. Die Reaktion wird in wäßrigem Medium durchgeführt, dem gegebenenfalls ein polares organisches Lösungsmittel, zum Beispiel ein Alkanol mit niedriger C-Zahl, zugesetzt werden kann. Die Reaktion wird in einem geschlossenem Reaktionsgefäß bei einer Temperatur von 80 bis 90°C und bei leichtem Überdruck von bis zu 3 bar vorgenommen. Die Reaktionszeit beträgt im allgemeinen 3 - 7 Stunden.

Die so erhaltenen quatärneren Ammoniumverbindungen, eignen sich zur Verminderung des Reibungswiderstandes von wäßrigen Medien. Sie werden zugegeber in Konzentration von 0,05 bis 5 Gew.-%, bevorzugt von 0,1 bis 1 Gew.-%, besonders bevorzugt von 0,2 bis 0,5 Gew.-%, wobei jedoch für jedes Tensid eine andere unter kritische Konzentrationsgrenze für eine ausreichende Wirksamkeit als SB existiert, die jedoch, wie weiter hinten beschrieben, durch einen einfachen Vorversuch bestimmt werden kann. Die Wirkung als SB ist außerdem abhängig von der Temperatur. Die erfindungsgemäß beanspruchten Verbindungen zeigen eine ausreichende Wirkung als SB verteilt über den Temperaturbereich von 0°C bis 120°C, wobei jedes einzelne Tensid jeweils eine Wirkungsbreite von ca. 40°C (± 10°C) besitzt. Die untere Temperaturgrenze ist bei allen Tensiden jeweils die Löslichkeitstemperatur in Wasser (Krafft-Punkt). Ist das Tensid jedoch in Lösung, so kann die Löslichkeitstemperatur einige Stunden bis Wochen um 5 bis 20°C unterschritten werden. Bei Verwendung der $C_{12}$-Alkylverbindungen eignen sich die genannten Tenside auch als SB für Temperaturen kleiner 0°C, wenn die Schmelztemperatur des Lösungsmittels Wasser durch Zumischung anderer Lösungsmittel, wie z. B. Ethylenglykol oder Isopropanol erniedrigt wird. Eine Absenkung der Schmelztemperatur durch Elektrolytzugabe ohne Verlust der Wirksamkeit als SB ist nur bedingt möglich. Für Temparaturen im Bereich von 80 bis 120°C sind besonders die folgenden Vebindungen geeignet.

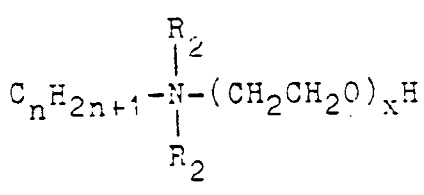

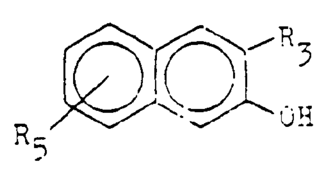

wobei

- n eine ganze Zahl von 20 bis 22 und
- x eine ganze oder gebrochene Zahl von 1 bis 1,5 ist, und
- $R_2$ für Methyl oder Ethyl,
- $R_3$ für $COO^-$ oder $SO_3^-$ und
- $R_5$ für H oder Methyl steht, wenn $R_3$ = $COO^-$ ist.

Weitere wurde gefunden, daß durch Erhöhung des pH-Wertes der wäßrigen Lösung auf pH-Werte über 9, vorzugsweise auf pH = 9,5 bis 11, durch Zugabe von NaOH oder anderer Basen oder durch Zugabe von $Na_2CO_3$ oder anderer Salze, die den pH-Werte erhöhen, die Wirksamkeit als SB entweder nicht beeinflußt wird, wie z. B. bei den Sulfonaten, oder wesentlich verbessert wird, wie z. B. bei den von Hydroxybenzoaten abgeleiteten Verbindungen. Auch eine Absenkung des pH-Wertes mit HCl oder anderen starken Säuren auf pH-Werte unter 4,5 führt zu der gleichen Beeinflussung der SB-Wirkung der Tenside.

Die Zugabe anderer Fremdelektrolyte führt von keiner Beeinflussung, wie z. B. bei den Sulfonaten, bis zu einer Verbesserung der Wirkung als SB, wie z. B. bei den von Hydroxybenzoaten abgeleiteten Verbindungen.

Als solche Fremdelektrolyte kommen in Frage beispielweise schwache Sauren wie Essigsäure oder Ameisensäure und Salze, die aus den folgenden Ionen gebildet werden: Alkali-, Erdalkali-, Übergansmetall-, Ammonium- oder Aluminium-Kationen; Halogenide, $ClO_3^{\ominus}$, $ClO_4^{\ominus}$, $BrO_3^{\ominus}$, $JO_3^{2\ominus}$, $S_2O_3^{2\ominus}$, $SO_4^{2\ominus}$, $S_2O_8^{2\ominus}$, $NO_2^{\ominus}$, $B_4O_7^{2\ominus}$, $NO_3^{\ominus}$, $PO_4^{3\ominus}$, $CO_3^{2\ominus}$, $CH_3COO^{\ominus}$, $C_2O_4^{2\ominus}$, $CN^{\ominus}$, $CrO_4^{2\ominus}$, $Cr_2O_7^{2\ominus}$.

Die Menge dieser Fremdelektrolyte, die man der wäßrigen Tensidlöung zugeben kann, nach oben begrenzt durch die Konzentration, bei der ein Aussalzeffekt für das Tensid auftritt, verbunden mit einer Abnahme oder dem völligen Verschwinden der Wirksamkeit als SB.

Die Wirkung der Fremdelektrolyte hängt auch ab von der Wertigkeit der Ionen und zwar verschiebt sich die Wirkung zu geringeren Konzentrationen gemäß folgendem Schema:

1-1-wertiger Elektrolyt 2-1 wertiger Elektrolyt

1-2-wertiger Elektrolyt 2-2-wertiger Elektrolyt

3-2-wertiger Elektrolyt 2-3-wertiger Elektrolyt.

Die Verbesserung der Wirksamkeit als Strömungbeschleuniger bei den von Hydroxybenzoaten abgeleiteten Verbindungen ist besonders effektiv bei Zugabe eines Salzes, das gleichzeitig den pH-Wert auf pH $\geqq$ 9,9 anhebt. So wirkt sich z. B. die Zugabe von $Na_2CO_3$ im Konzentrationsbereich von 0,1C bis 10C besonders positiver aus, wenn C die molare Konzentration des eingesetzten Tensids ist.

Anstelle der Zugabe von Salzen kann man auch so vorgehen, daß man das Halogensalz des kationischen Tensids $R_1K + Hal^-$, wie z. B. $[C_nH_{2n+1}N(CH_3)_2(CH_2CH_2O)_xH]Hal$ mit x = 1 bis 1,5 und mit Hal = Cl, Br, J im molaren Verhältnis 1 : 1 mit einem Alkalisalz des Anions NaA, wie z. B. Na-n-alkyl-1-sulfonat, Na-hydroxybenzoat und die davon abgeleiteten Säureanion oder wie z. B. Na-hydroxy-naphthoat als Strömungsbeschleuniger einsetzt. Die Wirkung ist dann gleich der Wirkung, die man mit den reinen Tensidsalzen unter Zusatz von Alkalihalogeniden erreicht. Auch Mischungen die vom molaren Verhältnis 1 : 1 abweichen, wie z. B. 1 : 2, zeigen noch Wirkung als SB.

Die maximale Wirksamkeit als Strömungsbeschleuniger ist auch von der Zeit abhängig, die verstrichen ist seit der Herstellung der wäßrigen Tensidlösungen. Die Tensidlösungen zeigen zwar bereits sofort nach dem Ansetzen der Lösungen eine Wirkung als Strömungsbeschleuniger, doch kann sich diese Wirkung während einer Woche noch deutlich ändern. Die Zeit, die benötigt wird, um eine optimale Wirkung zu erzielen, läßt sich für den Einzelfall unschwer durch einfache Versuche ermitteln. In den meisten Fällen wird die optimale Wirkung nach einer Woche erreicht. Eine Änderung oder Verbesserung der Wirkung tritt dann micht mehr ein.

Von einigen Tensiden, wie z. B. dem Hexadecylpyridiniumsalicylat ist bekannt (H. Hoffmann et al., Ber. Bunsenges. Phys. Chem., 85 (1981), 255), daß sie ab einer ganz bestimmten, für jedes Tensid charakteristischen Konzentrationen, der $CMC_{II}$, große nichtkugelförmige, meist stäbchenförmige Mizellen aus den einzelnen Tensidionen und Gegenionen aufbauen.

Überraschenderweise wurde nun gefunden, daß Tenside in wäßriger Lösung immer dann als Strömungsbeschleuniger wirksam sind, wenn sie für Konzentrationen größer der $CMC_{II}$ nichtkugelförmige, vorzugsweise stäbchenförmige Mizellen ausbilden. Nichtkugelförmige, vorzugsweise stäbchenförmige Mizellen liegen vor, wenn bei der Untersuchung der Tensidlösung mit Hilfe der Methode der elektrischen Doppelbrechung mit gepulstem, rechteckförmigem elektrischen Feld (E. Fredericq und C. Houssier, Electric Dichroism and Eletric Birefringence, Clarendon Press, Oxford 1973 und H. Hoffmann et al., Ber. Bunsenges. Phys. Chem., 85 (1981), 255) eine Meßsignal gefunden wird, aus dessen Abfall sich eine Relaxationszeit von $\tau \geqq 0,05\ \mu s$ bestimmen läßt.

Die untere Konzentrationsgrenze, ab der ein Tensid in wäßriger Lösung als Strömungsbeschleuniger wirksam ist, wird daher immer durch die $CMC_{II}$, vorzugsweise durch den 1,5-fachen Konzentrationswert der $CMC_{II}$, festgelegt. Die Bestimmung der $CMC_{II}$ ist z. B. durch Messung der elektrischen Leitfähigkeit der Tensidlösung in Abhängkeit von der Tensidkonzentration möglich, wie bei H. Hoffmann et al. (Ber. Bunsenges. Phys. Chem, 85 (1981), 255) beschrieben. Es zeigte sich, daß der Wert der $CMC_{II}$ temperaturabhängig ist und sich mit zunehmender Temperatur zu höheren Tensidkonzentrationen verschiebt. So beträgt z. B. für $C_{16}$DE-Sal die $CMC_{II}$ bei 40°C 250 ppm und bei 60°C 2000 ppm.

Zur Festlegung der Tensidkonzentration; die minimal notwendig ist, um eine ausreichende Wirkung als SB in einem bestimmten Temperaturbereich zu erzielen, ist die Bestimmung der $CMC_{II}$ bei der Anwendungstemperatur mit Hilfe der elektrischen Leitfähigkeit ein geeigneter Vorversuch.

Bei all den genannten Untersuchungen zeigte es sich bisher, daß nur Tenside, die Kationen der folgenden Formeln

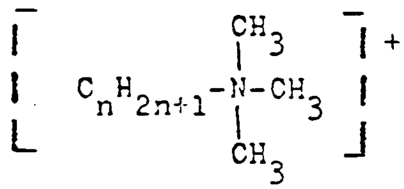

und

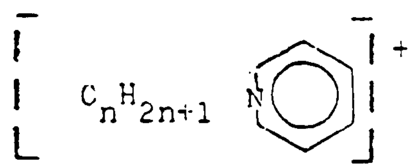

mit n = 12-24
enthalten, stäbchenförmige Mizellen aufbauen und als Strömungsbeschleuniger wirksam sind. Da aber schon kleine Variationen am quartären Stickoff zu einem Verlust der Wirksamkeit als SB führen, ist die Wirkung der hier beanspruchten Verbindungen, die eine zusätzliche hydrophile Gruppe enthalten, als SB völlig unvorhersehbar und überraschend.

Die Untersuchung der genannten Tenside auf ihre Wirksamkeit als SB erfolgt in den meisten Fällen in der üblichen Art, indem für die jeweilige wäßrige Lösung der Tenside der Druckabfall $\Delta P$ über die Strecke L bei Durchströmen eines Rohres mit dem Querschnitt d für verschiedene Strömungsgeschwindigkeiten u gemessen wurde. Aus diesen Werten lassen sich die dimensionslosen Größen, Reibungsbeiwert $\lambda$ und die Reynoldszahl Re, berechnen,

$$\lambda = \frac{2\,d}{\rho u'} \cdot \frac{\Delta P}{L}$$

$$RE = \frac{u\,d}{\gamma}$$

wobei $\rho$ die Dichte und $\gamma$ die kinematische Viskosität bedeuten. Üblicherweise werden für $\rho$ und $\gamma$ jeweils die entsprechenden Werte des reinen Lösungsmittels, Wasser, eingesetzt. Die so erhaltenen Werte $\lambda$, Re für die untersuchten Tensidlösungen wurden in der üblichen doppelt logaritmischen Auftragung $\gamma$ gegen Re mit den entsprechenden Werten für reines Wasser, wiedergegeben durch

$$1/\sqrt{\lambda} = 2 \log RE \sqrt{\lambda} - 0{,}8$$

verglichen. Eine Wirkung als SB oder auch Reibungsminderung liegt dann vor, wenn gilt:

$$\lambda H_2O - \lambda\, SB > 0,$$

bzw. die Größe der Reibungsminderung in Prozent berechnet sich nach:

$$\alpha = \%\ \text{Reibungsminderung} = \frac{\lambda\, H_2O \cdot \lambda\, Sb}{\lambda\, H_2O} \times 100$$

Wie aus Figur 1 ersichtlich ist, wirken die genannten Tensidlosüngen als SB in der Weise, daß die prozentuale Reibungsminderung mit ansteigender Reynoldszahl zunimmt, dann aber nach Überschreiten einer bestimmten Reynoldszahl, $Re_{max}$, mit maximaler prozentualer Reibungsminderung, sehr schnell wieder abnimmt. Der Grad der Wirksamkeit einer Tensidlösung als SB soll im folgenden Text durch die Größe von $Re_{max}$ gekennzeichnet werden; demnach besitzt einer Tensidlösung mit $Re_{max}$ = 20 000 eine bessere Wirksamkeit als SB als eine Tensidlösung mit $Re_{max}$ = 10 000. Der zugehörige, $\alpha$-Wert soll mit $\alpha_{max}$ gekennzeichnet werden. Die Untersuchungen der Tensidlösungen ergaben meistens nur dann reproduzierende Ergebnisse, wenn die wäßrigen Lösungen der Tensid-Salze vor den Messungen jeweils für ca 1 Woche bei den Meßtemperaturen aufbewahrt wurden. Die Lösungen zeigen zwar auch sofort nach dem Ansetzen eine Wirkung als Strömungsbeschleuniger, die sich jedoch im Verlauf einer Woche noch deutlich ändern kann.

Die so behandelten Tenside wurden einer Vielzahl von Tests unterzogen. So ergaben Dauerversuche über viele Tage, wie aus Beispiel 22 ersichtlich, daß bei den aufgeführten Tensiden keine Abnahme ihrer strömungsbeschleunigenden Wirkung durch mechanischen oder chemischen Abbau auftritt. Weiterhin zeigte sich, daß die Wirksamkeit als SB der genannten Tenside mit zunehmender Konzentration zunimmt; allerdings steigt auch

die Viskosität der Lösungen an, so daß die prozentuale Reibungsminderung bei kleineren Reynoldszahlen schlechter wird, wie aus der Zeichnung zu entnehmen ist.

Die durchgeführten Untersuchungen zeigen, daß sich die genannten Tensid-Salze als Strömungsbeschleuniger überall dort eignen, wo Wasser durch Rohrleitungen gepumpt wird, insbesondere aber dort, wo Wasser in einem Rohrleitungssystem ständig im Kreislauf umgepumpt wird, wie z. B. in Kühl- und Heizkreisläufen, da hier eine hohe Langzeitstabilität des SB, wie sie die genannten Tensid-Salze aufweisen, unbedingt erforderlich ist.

Die Zudosierung der Tensid-Salze in das die Rohrleitungen durchströmende Wasser kann sowohl in Form einer konzentrierten Tensidlösung (1 - 10 Gew.-%) erfolgen, als auch durch Zugabe der reinen kristallinen Tensid-Salze. Wegen des guten Durchmischungseffektes ist eine Zudosierung in das Rohrleitungssystem kurz vor einer Pumpe der günstigste Ort.

Herstellungsbeipiele:

Beispiel 1:

Hexadecyl-dimethyl-poly(oxethyl)$_{1-2}$-ammonium-(2-hydroxy-4-methoxy)benzoat

In einem 1 l-Rührautoklaven werden 68,5 g (0,25 mol) Hexadecyldimethylamin, 42,1 g (0,25 mol) 2-Hydroxy-4-methoxy-benzoesäure, 162 g Wasser und 32 g Isopropanol vorgelegt. Nach Erreichen einer Reaktionstemperatur von 80°C werden 22,1 g (0,5 Mol) Ethylenoxid so zugegeben, daß ein Druck von maximal 3 bar erreicht und eine Temperatur von 95°C nicht überschritten wird. Die Gesamtreaktionsdauer beträgt ca. 6 bis 7 Stunden bei ca. 80°C, wobei der Druck auf 0,2 bar absinkt. Die Analyse dieses Produktes weist einen Quaternisierungsgrad von 93 % auf.

Der Quaternisierungsgrad wird als Verhältnis aus der 2-Phasen-Titration der quaternären Ammoniumverbindung bei pH 1-2 bzw. pH 10 mit Natriumdodecylsulfat ermittelt.

Beispiel 2:

Es wurde eine Konzentrationsreihe von 750, 1500 und 3000 ppm von Hexadecyldimethyloxethylammonium-4-methoxysalicylat ($C_{16}$DE-4-methoxysalicylat) in E-Wasser angesetzt, indem die entsprechenden Gewichtsmengen von 0,075; 0,15 und 0,3 g von $C_{16}$DE-4-Methoxysalicylat auf 1000 g E-Wasser eingewogen wurden. Während des Auflösens wurden die Lösungen kurz auf 90°C erhitzt und nach dem Abkühlen auf Raumtemperatur (22°C) für 1 Woche ohne zu Rühren bei dieser Temperatur aufbewahrt.

Die Untersuchung auf Reibungsminderung erfolgt anschließend in einem Turbulenzrheometer (Polymer letters, 9, 851 (1971), Indem analog zu einer Spritze eine Flüssigkeitsmenge von 1,5 l mit Hilfe eines Kolbens durch das Meßrohr gedrückt wird. Die Bewegung des Kolbens wird während der Messung beschleunigt, so daß die gesamte Fließkurve wie in der Zeichnung dargestellt in einer Messung erfaßt wird. Der Durchmesser des Meßrohres beträgt 3 mm. Die Meßstrecke für Delta P 300 mm und die Einlaufstrecke 1200 mm.

In dieser Apparatur wurde dieselbe Konzentrationsreihe von $C_{16}$DE-4-Methoxysalicylat bei 22°C, 40°C, 55°C und 65°C gemessen, nachdem die Lösung zuvor jeweils für 1 Woche bei den entsprechenden Temperaturen aufbewahrt worden waren. Tabelle 1 faßt die Ergebnisse zusammen.

Beispiel 3:

Wäßrige Lösungen unterschiedlicher n-Hexadecyldimethyloxethylammonium-Salze ($C_{16}$-DE-Salz) und $C_{16}$-DE-2-oxy-3-naphthoat wurden durch Einwlegen der Salze $C_{16}$-DE-Cl und Na-1-nonat bzw. Na-2-oxy-3-naphthoat bzw. Na-1-heptansulfonat im molaren Mischungsverhältnis 1:1 hergestellt und wie in Beispiel 2 beschrieben, vorbehandelt.

Tabelle 2 faßt die Meßergebnisse zusammen, die bei den verschiedenen wäßrigen Lösungen unterschiedlicher Konzentration erhalten wurden.

**Tabelle 1**

Meßtemperatur 40° C

| Substanz | Konzentration/ppm | $Re_{max}$ | $\alpha_{max}$ |
|---|---|---|---|
| $C_{16}$DE-4-methoxyl-salicylat | 750 | 5000 ± 500 | 51 ± 3 |
| | 1500 | 7900 ± 800 | 61 ± 3 |
| | 3000 | 10200 ± 1000 | 62 ± 3 |
| $C_{16}$DE-caprylat | 10000 | 5300 ± 500 | 47 ± 3 |

**Tabelle 2**

| Substanz | Temperatur °C | Konzentration ppm | $Re_{max}$ | $\alpha_{max}$ |
|---|---|---|---|---|
| $C_{16}$DE-2-oxi-3 naphthoat | 60 | 750 | 24200 ± 2400 | 72 ± 4 |
| | 60 | 1500 | 32800 ± 3300 | 72 ± 4 |
| | 85 | 2000 | 31200 ± 3100 | 73 ± 4 |

## Patentansprüche

1. Quartäre Ammoniumverbindungen der Formel

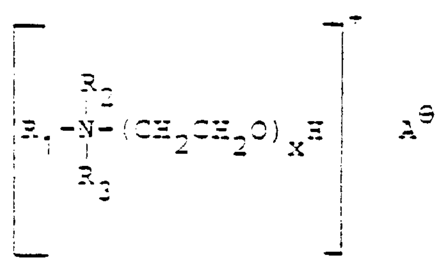

worin $R_1$ einen Alkyl- oder Alkenylrest mit 12 bis 22 C-Atomen; $R_2$ und $R_3$ gleich oder verschieden, einen Alkylrest mit 1 bis 4 C-Atomen; x eine ganze oder gebrochene Zahl von 1 bis 3; und $A^-$ ein Anion der Formel

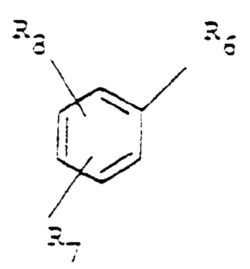

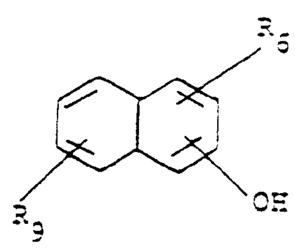

wobei $R_6$-$COO^-$ oder -$SO_3^-$,
wenn $R_6$ $SO_3^-$, dann ist $R_7$ Wasserstoff oder Hydroxy in den Stellungen 2 und 3 zu $R_6$ und $R_8$ $C_1$-$C_5$-Alkyl, $C_2$-$C_5$-Alkenyl oder $C_1$-$C_5$-Alkoxy in den Stellungen 3, 4 oder 5 zu $R_6$ oder wenn $R_6$ $COO^-$ dann ist $R_7$ Wasserstoff und $R_8$ $C_1$-$C_5$-Alkoxy in den Stellungen 3,4 oder 5 zu $R_6$ oder dann ist $R_7$ Hydroxy in den Stellungen 2 und 3 zu $R_6$ und $R_8$ $C_1$-$C_5$-Alkyl, $C_2$-$C_5$-Alkenyl oder $C_1$-$C_5$-Alkoxy in den Stellungen 3,4 oder 5 zu $R_6$, wobei $R_9$ Wasserstoff oder Methyl bedeutet, ausgenommen die Verbindungen mit $A^-$ = Tosylat.

2. Verbindungen der Formel

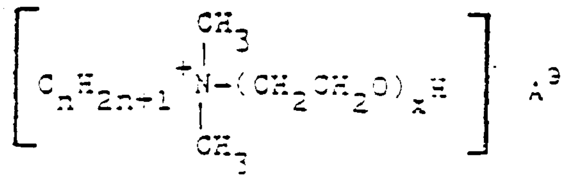

wobei x eine ganze oder gebrochene Zahl von 1 bis 1,5 und n eine Zahl von 12 bis 24 und $A^-$ ein Anion aus der Gruppe

a) Anionen der Formel

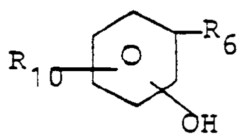

mit $R_6 = SO_3^-$ oder $COO^-$ und $R_{10} = C_1$-$C_4$-Alkyl oder $C_1$-$C_4$-Alkoxy

b) 2-Hydroxy-1-naphthoat, 3-(oder 4)-Hydroxy-2-naphthoat oder die entsprechenden Naphtholsulfonsäure-Anionen bedeuten.

3. Verfahren zur Herstellung der Verbindungen nach Anspruch 1 und 2, dadurch gekennzeichnet, daß man ein Amin der Formel

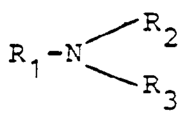

gleichzeitig mit Ethylenoxid und einer zur Neutralisation equivalenten Menge einer Säure der Formel

A - H

umsetzt.

4. Verfahren nach Anspruch 3, dadurch gekennzeichnet, daß man das Amin mit der Säure neutralisiert und dann mit Ethylenoxid umsetzt.

5. Verwendung der Verbindungen nach Anspruch 1 als Strömungsbeschleuniger.

## Claims

1. A quaternary ammonium compound of the formula

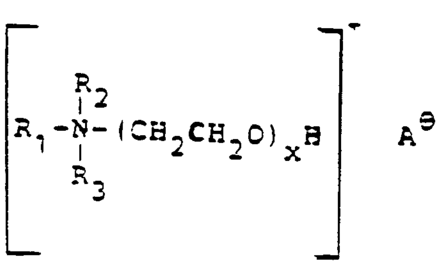

in which $R_1$ is an alkyl or alkenyl radical with 12 to 22 carbon atoms; $R_2$ and $R_3$ are identical or different and are an alkyl radical with 1 to 4 carbon atoms; x is an integral or non-integral number from 1 to 3; and $A^-$ is an anion of the formula

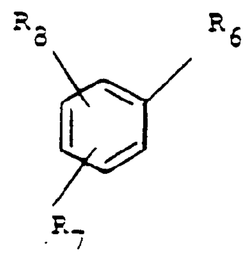

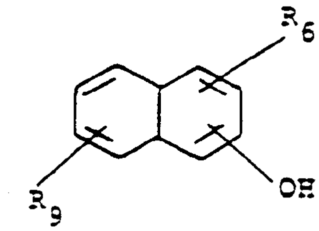

in which $R_6$ is $-COO^-$ or $-SO_3^-$,

$R_7$ if $R_6$ is $SO_3^-$, hydrogen or hydroxyl in positions 2 and 3 relative to $R_6$, and $R_8$ is $C_1$-$C_5$-alkyl, $C_2$-$C_5$-alkenyl or $C_1$-$C_5$-alkoxy in positions 3, 4 or 5 relative to $R_6$, or if $R_6$ is $COO^-$, $R_7$ is hydrogen and $R_8$ is $C_1$-$C_5$-alkoxy in positions 3, 4 or 5 relative to $R_6$ or $R_7$ is hydroxyl in positions 2 and 3 relative to $R_6$, and $R_8$ is $C_1$-$C_5$-alkyl, $C_2$-$C_5$-alkenyl or $C_1$-$C_5$-alkoxy in positions 3, 4 or 5 relative to $R_6$, $R_9$ being hydrogen or methyl, the compounds where $A^-$ = tosylate being excluded.

2. A compound of the formula

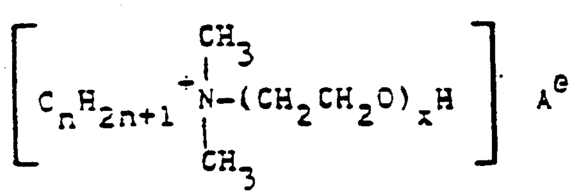

in which x is an integral or non-integral number from 1 to 1.5, n is a number from 12 to 24 and $A^-$ is an anion from the group

a) an anion of the formula

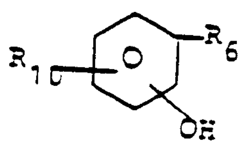

where $R_6 = SO_3^-$ or $COO^-$ and $R_{10}$ = $C_1$-$C_4$-alkyl or $C_1$-$C_4$-alkoxy, and

b) 2-hydroxy-1-naphthoate, 3-(or 4)-hydroxy-2-naphthoate or the corresponding naphtholsulfonic acid anions.

3. A process for the preparation of a compound as claimed in claim 1 and 2, which comprises reacting an amine of the formula

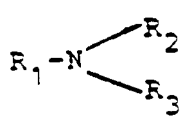

simultaneously with ethylene oxide and an acid of the formula

A - H

in an amount equivalent for neutralization.

4. The process as claimed in claim 3, wherein the amine is neutralized with the acid and then reacted with ethylene oxide.

5. The use of a compound as claimed in claim 1 as a drag-reducing agent.

## Revendications

1. Composés d'ammonium quaternaire répondant à la formule :

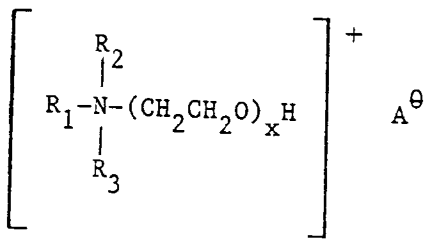

dans laquelle $R_1$ représente un reste alkyle ayant 12 à 22 atomes de carbone ; $R_2$ et $R_3$, identiques ou différents, représentent chacun un reste alkyle ayant 1 à 4 atomes de carbone ; x est un nombre entier ou fractionnaire valant 1 à 3 ; et $A^{\theta}$ représente un anion répondant à l'une des formules :

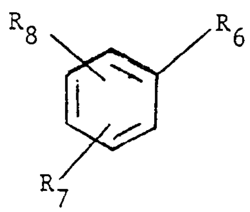

et

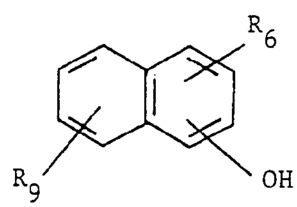

dans lesquelles $R_6$ représente $-COO^-$ ou $-SO_3^-$,
dans le cas où $R_6$ représente $-SO_3^-$ : $R_7$ représente l'hydrogène ou un hydroxy aux positions 2 et 3 par rapport à $R_6$ et $R_8$ représente un alkyle en $C_1$-$C_5$, un alcényle en $C_2$-$C_5$ ou un alcoxy en $C_1$-$C_5$ aux positions 3, 4 ou 5 par rapport à $R_6$, et dans le cas où $R_6$ représente $-COO^-$ : $R_7$ représente l'hydrogène et $R_6$ représente un alcoxy en $C_1$-$C_5$ aux positions 3, 4 ou 5 par rapport à $R_6$, ou $R_7$ représente un hydroxy aux positions 2 et 3 par rapport à $R_6$ et $R_8$ représente un alkyle en $C_1$-$C_5$, un alcényle en $C_2$-$C_5$ ou un alcoxy en $C_1$-$C_5$ aux positions 3, 4 ou 5 par rapport à $R_6$, et $R_9$ représente l'hydrogène ou un méthyle , sauf les composés pour lesquels $A^{\theta}$ représente un radical tosylate.

2. Composés répondant à la formule :

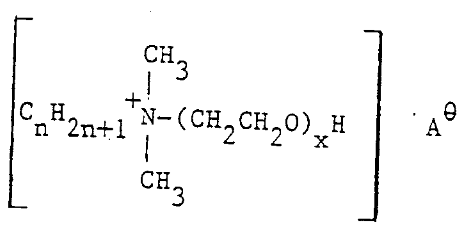

dans laquelle x est un nombre entier ou fractionnaire valant 1 à 1,5, n est un nombre valant 12 à 24 et $A^{\theta}$ est un anion pris dans l'ensemble constitué par :

a) les anions répondant à la formule :

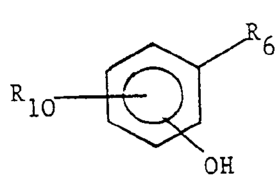

dans laquelle $R_6$ représente un groupe $CO_3^-$ ou $COO^-$ et $R_{10}$ représente un groupe alkyle en $C_1$-$C_4$ ou

alcoxy en $C_1$-$C_4$, et

b) les anions hydroxy-2 naphtoate-1, hydroxy-3 naphtoate-2, hydroxy-4 naphtoate-2 et les anions hydroxy-naphtalène-sulfoniques correspondants.

3. procédé pour préparer les composés selon l'une des revendications 1 et 2, procédé caractérisé en ce qu'on fait réagir une amine de formule :

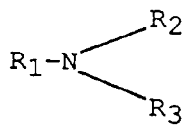

en même temps avec l'oxyde d'éthylène et une quantité d'un acide de formule :

A - H

équivalente pour la neutralisation.

4. Procédé selon la revendication 3 caractérisé en ce qu'on neutralise l'amine avec l'acide, puis on fait réagir avec l'oxyde d'éthylène.

5. Application des composés selon la revendication 1 comme accélérateurs d'écoulement.

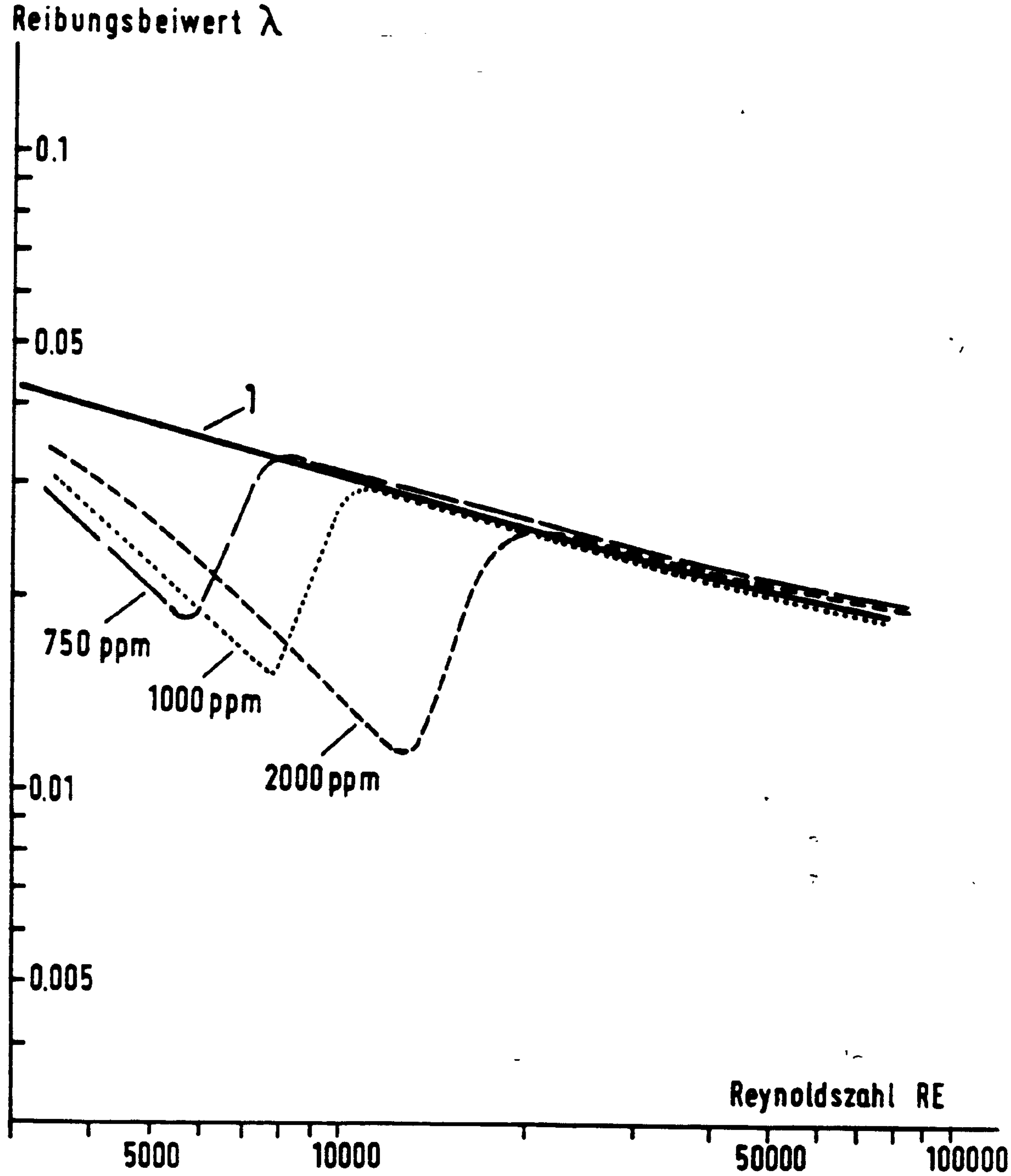
Reibungsbeiwert λ
0.1
0.05
0.01
0.005
1
750 ppm
1000 ppm
2000 ppm
Reynoldszahl RE
5000
10000
50000
100000